# FASCICULE DE BREVET EUROPEEN

(11) **EP 1 890 990 B1**
(45) Date de publication et mention de la délivrance du brevet: **01.08.2012**
(21) Numéro de dépôt: 06764760.2
(22) Date de dépôt: 09.06.2006
(51) Int. Cl.: C07C 51/235, C07C 51/43, C07C 51/48, C07C 59/105, C07C 51/215, C07C 55/14, C07C 55/02

(54) **PROCEDE DE FABRICATION D'ACIDES CARBOXYLIQUES**
VERFAHREN ZUR HERSTELLUNG VON CARBONSÄUREN
METHOD FOR MAKING CARBOXYLIC ACIDS

(30) Priorité: 17.06.2005 FR 0506160
(43) Date de publication de la demande: 27.02.2008
(73) Titulaire: RHODIA CHIMIE, 93300 Aubervilliers (FR)
(72) Inventeur: BONNET, Didier, F-69004 Lyon (FR); PETROFF SAINT-ARROMA, Romain, F-75009 Paris (FR); RIGHINI, Sébastien, F-69004 Lyon (FR); IRELAND, Tania, F-69007 Lyon (FR); SIMONATO, Jean-Pierre, F-38360 Sassenage (FR)
(74) Mandataire: Esson, Jean-Pierre
(86) Numéro de dépôt international: PCT/FR2006/001308
(87) Numéro de publication internationale: WO 2006/136674

(56) Documents cités:
- EP-A- 1 101 753
- FR-A- 2 775 685
- FR-A- 2 791 667
- FR-A- 2 806 079
- FR-A- 2 828 194
- US-A- 5 294 739

## Description

La présente invention concerne un procédé de fabrication d'acides carboxyliques.

Elle se rapporte plus particulièrement à un procédé de fabrication d'acides carboxyliques par oxydation d'hydrocarbure par l'oxygène ou un gaz contenant de l'oxygène, et encore plus particulièrement à l'oxydation du cyclohexane en acide adipique.

L'acide adipique est un composé chimique important utilisé dans de nombreux domaines. Ainsi, l'acide adipique peut être utilisé comme additif dans de nombreux produits tant dans le domaine alimentaire que les bétons. Toutefois, une des utilisations les plus importantes est son application comme monomère dans la fabrication de polymères dont les polyuréthanes et les polyamides.

Plusieurs procédés de fabrication d'acide adipique ont été proposés. Un des plus importants, utilisé industriellement à grande échelle, consiste à oxyder en une ou deux étape(s) le cyclohexane en un mélange de cyclohexanol/cyclohexanone par un gaz contenant de l'oxygène ou par l'oxygène. Après extraction et purification du mélange cyclohexanol/cyclohexanone, ces composés sont oxydés notamment en acide adipique par l'acide nitrique.

Toutefois, ce procédé présente un inconvénient majeur lié à la formation de vapeur nitreuse.

De nombreux travaux ont été effectués pour la mise au point d'un procédé d'oxydation par l'oxygène ou un gaz contenant de l'oxygène, d'hydrocarbures permettant d'obtenir directement les acides carboxyliques, principalement l'acide adipique.

Ces procédés sont décrits notamment dans les brevets FR2761984, FR2791667, US 5294739.

Généralement, la réaction est réalisée en milieu solvant, le solvant étant un acide monocarboxylique comme l'acide acétique. D'autres solvants ont été proposés comme les acides carboxyliques à caractère lipophile décrits dans le brevet FR 2806079.

De nombreux brevets ont décrit les conditions opératoires de cette réaction ainsi que les différentes étapes pour extraire les acides formés, les purifier et également recycler l'hydrocarbure non oxydé ainsi que le catalyseur.

Toutefois, dans cette réaction d'oxydation, il se forme des sous-produits qui peuvent diminuer de manière plus ou moins importante le rendement du procédé. Parmi ceux-ci, certains, tels que les alcools, réagissent avec les acides formés pour donner des esters qui doivent être extraits du milieu réactionnel pour éviter leur accumulation ou la production d'impuretés indésirables et difficiles à séparer des acides formés.

FR 2 775 685 décrit un procédé de séparation et de purification de l'acide adipique issu d'une réaction d'oxydation du cyclohexane. Après séparation du catalyseur par distillation, l'acide adipique est purifié par un traitement d'oxydation en solution aqueuse.

D'autres produits intermédiaires d'oxydation tels que les composés α, ω-hydroxycarboxyliques sont également gênants s'ils ne sont pas éliminés du milieu réactionnel ou transformés. En effet, ces composés sont souvent difficilement séparables des diacides, rendant difficile l'obtention de diacide pur, notamment présentant le degré de pureté requis pour l'utilisation comme monomère dans la fabrication des polyamides.

Il est important pour l'économie du procédé et également pour une production de diacides à degré de pureté élevée, de diminuer la concentration en sous produits dans le milieu réactionnel et notamment dans les diacides récupérés.

Un des buts de la présente invention est de proposer un procédé de fabrication de diacides permettant d'éliminer, extraire ou transformer les sous-produits résultant de la réaction d'oxydation.

A cet effet, l'invention propose un procédé de fabrication de diacides carboxyliques par oxydation d'un hydrocarbure cycloaliphatique avec de l'oxygène moléculaire ou un gaz contenant de l'oxygène moléculaire, en présence d'un solvant.

Selon l'invention, le procédé comprend une étape d'oxydation de l'hydrocarbure et au moins une étape pour extraire les acides dicarboxyliques formés du milieu réactionnel et éventuellement recycler l'hydrocarbure non transformé avec des sous-produits d'oxydation tels que les alcools et cétones qui peuvent se former.

Le procédé de l'invention comprend également une étape de transformation élimination ou extraction des composés α, ω-hydroxycarboxyliques formés au cours de l'étape d'oxydation.

Cette étape de transformation, élimination ou extraction des composés α, w-hydroxycarboxyliques consiste à soumettre le milieu contenant ces composés à une oxydation pour les transformer en diacides. Cette réaction d'oxydation est réalisée éventuellement en présence d'un catalyseur comprenant comme élément catalytiquement actif un métal ou composé métallique choisi dans le groupe comprenant Cu, Ag, Au, Mg, Ca, Sr, Ba, Zn, Cd, Hg, Al, Sc, In, TI, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, les lanthanides comme Ce et les combinaisons de ceux-ci, préférentiellement les métaux précieux tels que le platine, l'or, l'argent, le ruthénium, le rhénium, le palladium ou leurs mélanges. Avantageusement, ce métal ou composé métallique catalytiquement actif est déposé, imprégné ou greffé sur un support poreux tel que le noir de carbone, l'alumine, les zéolithes, la silice, le graphite et plus généralement les supports utilisés dans le domaine de la catalyse

Le catalyseur préféré de l'invention est notamment un catalyseur comprenant un composé du platine déposé sur du noir de carbone.

La réaction d'oxydation des composés α, ω-hydroxycarboxyliques est avantageusement réalisée à une température comprise entre 50 et 150°C.

L'agent d'oxydation convenable pour cette étape est avantageusement l'oxygène moléculaire ou un gaz contenant de l'oxygène moléculaire. Il est également possible d'utiliser d'autres agents d'oxydation tels que l'eau oxygénée, l'ozone, l'acide nitrique.

Selon un premier mode de réalisation de l'invention, l'étape de transformation, élimination ou extraction des composés hydroxycarboxyliques est réalisée sur le milieu sortant du réacteur d'oxydation préalablement à la séparation des diacides formés et de l'hydrocarbure n'ayant pas réagi, c'est à dire en présence de la phase organique

Selon un deuxième mode de réalisation de l'invention, l'étape de transformation des composés hydroxycarboxyliques est réalisée sur le milieu contenant les diacides formés après extraction de ceux-ci du milieu réactionnel d'oxydation, ou après cristallisation des diacides, sur les eaux mères de cristallisation, c'est-à-dire sur un milieu constitué par une phase aqueuse.

Ainsi, dans le premier mode de réalisation de l'invention, un catalyseur d'oxydation homogène ou hétérogène, est ajouté dans le milieu réactionnel, soit dans le réacteur d'oxydation après la fin de la réaction d'oxydation de l'hydrocarbure, soit dans un ou plusieurs réacteurs d'oxydation distincts dans lesquels est alimenté le milieu réactionnel. Dans ce mode de réalisation, le catalyseur utilisé est avantageusement un catalyseur métallique homogène ou un mélange de catalyseurs homogènes. La condition de température est définie et par exemple comprise entre 50 et 150°C.

L'oxydant est avantageusement l'oxygène, ou un gaz contenant de l'oxygène, comme l'air, par exemple. Dans ce cas, la pression partielle en oxygène est avantageusement entre 0.1 à 30 bar.

Dans le second mode de réalisation de l'invention, l'oxydation des composés α, w-hydroxycarboxyliques est réalisée en milieu aqueux soit en absence de catalyseur soit en présence d'un catalyseur tel que défini ci-après. Avantageusement, le catalyseur est un catalyseur hétérogène et l'oxydant est l'oxygène, un gaz contenant l'oxygène, l'acide nitrique, l'eau oxygénée, l'ozone, par exemple.

Le procédé de l'invention s'applique notamment à l'oxydation du cyclohexane pour obtenir l'acide adipique. Il peut également s'appliquer à l'oxydation d'autres hydrocarbures tels que le cyclododécane.

La réaction d'oxydation de l'hydrocarbure, par exemple du cyclohexane, est généralement mise en oeuvre en présence d'un solvant. Ce solvant peut être de nature très variée dans la mesure où il n'est pas oxydable dans les conditions réactionnelles. Il peut être notamment choisi parmi les solvants protiques polaires et les solvants aprotiques polaires. Comme solvants protiques polaires, on peut citer par exemple, les acides carboxyliques ne possédant que des atomes d'hydrogène primaires ou secondaires, en particulier les acides aliphatiques ayant de 2 à 9 atomes de carbone tel que l'acide acétique, les acides perfluoroalkylcarboxyliques tel que l'acide trifluoroacétique, les alcools tels que le tertiobutanol, les hydrocarbures halogénés tel que le dichlorométhane, les cétones telles que l'acétone. Comme solvants aprotiques polaires, on peut citer par exemple les esters d'alkyle inférieur (=radical alkyle ayant de 1 à 4 atomes de carbone) d'acides carboxyliques, en particulier des acides carboxyliques aliphatiques ayant de 2 à 9 atomes de carbone ou des acides perfluoroalkylcarboxyliques, la tétraméthylènesulfone (ou sulfolane), l'acétonitrile, ou le benzonitrile.

Le solvant peut également être choisi parmi les acides carboxyliques à caractère lipophile.

Par composé acide lipophile convenable pour l'invention, on entend les composés acides aromatiques, aliphatiques, arylaliphatiques ou alkylaromatiques comprenant au moins 6 atomes de carbone, pouvant comprendre plusieurs fonctions acides et présentant une faible solubilité dans l'eau, c'est à dire une solubilité inférieure à 10 % en poids à température ambiante (10°C - 30°C).

Comme composé organique lipophile on peut citer par exemple, les acides hexanoïque, heptanoïque, octanoïque, éthyl-2 hexanoïque, nonanoïque, décanoïque, undécanoïque, dodécanoïque, stéarique (octadécanoïque) et leurs dérivés perméthylés (substitution totale des hydrogènes des groupes méthylènes par le groupe méthyle), l'acide 2-octadécylsuccinique, 3,5-ditertiobutylbenzoïque, 4-tertiobutylbenzoïque, 4-octylbenzoïque, l'hydrogénoorthophtalate de tertiobutyle, les acides naphténiques ou anthracéniques substitués par des groupements alkyles, de préférence de type tertiobutyle, les dérivés substitués des acides phtaliques, les diacides gras comme le dimère d'acide gras. On peut également citer les acides appartenant aux familles précédentes et porteurs de différents substituants électrodonneurs (groupements avec hétéroatome du type O ou N) ou électroaccepteurs (halogènes, sulfonimides, groupements nitro, sulfonato ou analogues). Les acides aromatiques substitués sont préférés.

De manière générale, le solvant est choisi pour obtenir avantageusement une phase homogène dans les conditions de température et de pression auxquelles est mise en oeuvre la réaction d'oxydation. Pour cela, il est avantageux que la solubilité du solvant dans l'hydrocarbure ou le milieu réactionnel soit au moins supérieure à 2 % en poids, et qu'au moins une phase liquide homogène comprenant au moins une partie des hydrocarbures à oxyder et une partie du solvant soit formée.

Avantageusement, le solvant est choisi parmi ceux qui sont peu solubles dans l'eau, c'est à dire qui présentent une solubilité dans l'eau inférieure à 10 % en poids à température ambiante (10-30°C).

Toutefois, il est possible sans sortir du cadre de l'invention, d'utiliser un solvant présentant une solubilité dans l'eau supérieure à celle indiquée précédemment si le coefficient de partage de ce composé entre la ou les phases organiques du milieu réactionnel constituées essentiellement par l'hydrocarbure à oxyder, les intermédiaires d'oxydation et la phase non organique comprenant l'eau formée pendant la réaction d'oxydation permet d'obtenir une concentration du solvant dans ladite phase aqueuse inférieure à 10 % en poids.

L'oxydation est réalisée, en général, en présence d'un catalyseur. Ce catalyseur comprend avantageusement un élément métallique choisi dans le groupe comprenant Cu, Ag, Au, Mg, Ca, Sr, Ba, Zn, Cd, Hg, Al, Sc, In, Tl, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, les lanthanides comme Ce et les combinaisons de ceux-ci.

Ces éléments catalytiques sont mis en oeuvre soit sous forme de composés avantageusement au moins partiellement solubles dans le milieu liquide d'oxydation aux conditions de mise en oeuvre de la réaction d'oxydation, soit supportés, absorbés ou liés à un support inerte tel que silice, alumine, par exemple.

Le catalyseur est de préférence, notamment aux conditions de mise en oeuvre de la réaction d'oxydation :
- soit soluble dans l'hydrocarbure à oxyder,
- soit soluble dans le solvant,
- soit soluble dans le mélange hydrocarbure/solvant formant une phase liquide homogène aux conditions de mise en oeuvre de la réaction.

Selon un mode de réalisation préféré de l'invention, le catalyseur utilisé est soluble dans l'un de ces milieux à température ambiante ou à la température de recyclage de ces milieux dans une nouvelle oxydation.

Par le terme soluble, on entend que le catalyseur soit au moins partiellement soluble dans le milieu considéré.

Dans le cas d'une catalyse hétérogène, les éléments métalliques catalytiquement actifs sont supportés ou incorporés dans une matrice minérale micro ou mésoporeuse ou dans une matrice polymérique ou sont sous forme de complexes organométalliques greffés sur un support organique ou minéral. Par incorporé, on entend que le métal est un élément du support ou que l'on travaille avec des complexes stériquement piégés dans des structures poreuses dans les conditions de l'oxydation.

Dans un mode de réalisation préféré de l'invention, le catalyseur homogène ou hétérogène est constitué de sels ou de complexes de métaux des groupes IVb (groupe du Ti), Vb (groupe du V), Vlb (groupe du Cr), VIIb (groupe du Mn), VIII (groupe du Fe ou Co ou Ni), Ib (groupe du Cu) et cérium, seuls ou en mélange. Les éléments préférés sont, en particulier, Mn et/ou Co en association avec un ou plusieurs autres éléments métalliques tel que par exemple Zr, Hf, Ce, Hf, Fe. Les concentrations en métal dans le milieu liquide d'oxydation varient entre 0,00001 et 5 % (% poids), de préférence entre 0,001 % et 2 %.

Par ailleurs, la concentration en solvant dans le milieu réactionnel est avantageusement déterminée pour avoir un rapport molaire entre le nombre de molécules de solvant et le nombre d'atome de métal catalytique compris entre 0,5 et 100 000, de préférence entre 1 et 5000.

La concentration en solvant dans le milieu liquide d'oxydation peut varier dans de larges limites. Ainsi, elle peut être comprise entre 1 et 99 % en poids par rapport au poids total du milieu liquide, plus avantageusement elle peut être comprise entre 2 et 50 % en poids du milieu liquide.

Il est également possible, sans pour cela sortir du cadre de l'invention, d'utiliser le solvant en association avec un autre composé qui peut notamment avoir comme effet d'améliorer la productivité et/ou la sélectivité de la réaction d'oxydation en acide adipique, et notamment la solubilisation de l'oxygène.

Comme exemples de tels composés, on peut citer, en particulier, les nitriles, les composés hydroxyimides, les composés halogénés, plus avantageusement les composés fluorés. Comme composés plus particulièrement convenables, on peut citer les nitriles comme l'acétonitrile, le benzonitrile, les imides appartenant à la famille décrite dans la demande brevet EP 0824962, et plus particulièrement la N-hydroxysuccinimide (NHS) ou la N-hydroxyphtalimide (NHPI), les dérivés halogénés comme le dichlorométhane, les composés fluorés comme :
- Hydrocarbures aliphatiques fluorés ou perfluorés cycliques ou acycliques,
- hydrocarbures fluorés aromatiques tels le perfluorotoluène, perfluorométhylcyclohexane, perfluoroheptane, perfluorooctane, perfluorononane, perfluorodécaline, perfluorométhyldécaline, α, α, α-trifluorotoluène, 1,3-bis (méthyl trifluoro) benzène).
- Esters perfluorés ou fluorés tels que perfluorooctanoates d'alkyle, perfluoronanoates d'alkyle.
- Cétones fluorées ou perfluorées telles que acétone perfluorée.
- Alcools fluorés ou perfluorés tels que hexanol, octanol, nonanol, décanol perfluorés, t-butanol perfluoré, isopropanol perfluoré, hexafluoro-1,1,1,3,3,3-propanol-2.
- Nitriles fluorés ou perfluorés tels que acétonitrile perfluoré.
- Acides fluorés ou perfluorés tels que acides trifluorométhylbenzoïque, acide pentafluorobenzoïque, acide hexanoïque, heptanoïque, octanoïque, nonanoïque perfluorés, acide adipique perfluoré.
- Halogénures fluorés ou perfluorés tels que iodo octane perfluoré, bromooctane perfluoré.
- Amines fluorées ou perfluorées tels que tripropylamine perfluorée, tributylamine perfluorée, tripentylamine perfluorée.

L'invention s'applique plus particulièrement à l'oxydation de composés cycloaliphatiques tels que le cyclohexane, le cyclododécane en diacides linéaires correspondants, l'acide adipique, l'acide dodécanedioïque.

Selon un mode de réalisation préféré de l'invention, elle concerne l'oxydation directe du cyclohexane en acide adipique, par l'oxygène ou un gaz contenant de l'oxygène, en milieu liquide et en présence d'un catalyseur au manganèse ou une association manganèse/cobalt.

La réaction d'oxydation est mise en oeuvre à une température comprise entre 50°C et 200°C, de préférence entre 70°C et 180°C. Elle peut être réalisée sous pression atmosphérique. Toutefois, elle est généralement mise en oeuvre sous pression pour maintenir les composants du milieu réactionnel sous forme liquide. La pression peut être comprise entre 10kpa (0,1 bar) et 20000 kpa (200 bars), de préférence entre 100 kpa (1 bar) et 10000 kpa (100 bars).

L'oxygène utilisé peut être sous forme pure ou en mélange avec un gaz inerte tel que l'azote ou l'hélium. On peut également utiliser de l'air plus ou moins enrichi en oxygène. La quantité d'oxygène alimentée dans le milieu est avantageusement comprise entre 1 et 1000 moles par mole de composés à oxyder.

Le procédé d'oxydation peut être réalisé de manière continue ou selon un procédé discontinu. Avantageusement, le milieu réactionnel liquide sorti du réacteur est traité selon des procédés connus permettant d'une part de séparer et récupérer les diacides produits et d'autre part de recycler les composés organiques non oxydés ou partiellement oxydés comme le cyclohexane, le cyclohexanol et/ou la cyclohexanone. Il est avantageux de mettre en oeuvre également un composé initiateur de la réaction d'oxydation, tel que par exemple une cétone, un alcool, un aldéhyde ou un hydroperoxyde. La cyclohexanone, le cyclohexanol et l'hydroperoxyde de cyclohexyle qui sont des intermédiaires réactionnels dans le cas de l'oxydation du cyclohexane, sont tout particulièrement indiqués. Généralement l'initiateur représente de 0,01 % à 20 % en poids du poids du mélange réactionnel mis en oeuvre, sans que ces proportions aient une valeur critique. L'initiateur est surtout utile lors du démarrage de l'oxydation. Il peut être introduit dès le début de la réaction.

L'oxydation peut également être mise en oeuvre en présence d'eau introduite dès le stade initial du procédé.

Comme indiqué ci-dessus, le mélange réactionnel issu de l'oxydation est soumis à différentes opérations de séparation de certains de ses constituants pour, par exemple, permettre leur recyclage au niveau de l'oxydation et la récupération des acides produits.

Selon un premier mode de réalisation de l'invention, le milieu sortant du réacteur d'oxydation est directement soumis, à une seconde étape d'oxydation, en présence d'un catalyseur métallique homogène ou hétérogène. Les conditions de température et de pression peuvent être semblables ou différentes des conditions utilisées dans l'étape d'oxydation de l'hydrocarbure. L'oxydant utilisé peut être l'oxygène, un gaz contenant l'oxygène, l'eau oxygénée, l'ozone, un hydroperoxyde organique ou analogue, par exemple. Dans cette étape d'oxydation, le composé α, ω-hydroxycarboxylique formé, tel que l'acide hydroxycaproïque dans le cas de l'oxydation du cyclohexane est transformé en acide dicarboxylique. Comme indiqué précédemment cette étape est mis en oeuvre soit dans le réacteur d'oxydation soit dans un ou plusieurs réacteurs supplémentaires.

Le catalyseur est avantageusement un catalyseur homogène constitué par au moins un composé d'un métal choisi dans le groupe comprenant Cu, Ag, Au, Mg, Ca, Sr, Ba, Zn, Cd, Hg, Al, Sc, In, TI, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, les lanthanides comme Ce et les combinaisons de ceux-ci.

Il est également possible d'utiliser un catalyseur hétérogène comprenant comme phase catalytique un des composés métalliques ci-dessus.

En sortie de cette étape, le mélange réactionnel est refroidi et décanté en au moins deux phases liquides : une ou plusieurs phases organiques contenant essentiellement l'hydrocarbure n'ayant pas réagi, éventuellement le solvant et certains intermédiaires d'oxydation tels que les alcools et les cétones, et une phase aqueuse contenant les diacides formés au cours de l'oxydation de l'hydrocarbure et au cours de l'étape de transformation des composés α, ω-hydroxycarboxyliques.

Avantageusement, la phase organique est lavée plusieurs fois avec de l'eau ou une solution aqueuse acide pour extraire la quantité maximale de diacides carboxyliques.

La phase organique qui contient l'hydrocarbure (cyclohexane) non oxydé ainsi que certains composés intermédiaires d'oxydation tels que la cyclohexanone, le cyclohexanol, est recyclée, avantageusement, à J'étape d'oxydation de l'hydrocarbure.

Dans le cas où le solvant acide est un solvant à caractère lipophile, ce solvant est présent dans la phase organique car il est insoluble dans l'eau. Il est, de ce fait, recyclé à l'étape d'oxydation avec le cyclohexane non oxydé. Ce recyclage du solvant intervient notamment quand le solvant est choisi parmi les acides aromatiques substitués ou non tels que l'acide tertiobutyl benzoïque.

Les diacides formés, notamment l'acide adipique, sont récupérés à partir de la phase aqueuse, par exemple, par cristallisation.

Les acides ainsi récupérés sont avantageusement purifiés selon les techniques habituelles et décrites dans de nombreux documents. Parmi les procédés de purification, la purification par cristallisation dans différents solvants tel que l'eau, solution aqueuse d'acide acétique, alcools, sont préférés. Des procédés de purification sont notamment décrits dans les brevets français n° 2749299 et 2749300.

De même, si le catalyseur d'oxydation de l'hydrocarbure n'est pas recyclé entièrement avec la phase organique, et est en partie ou totalement extrait avec la phase aqueuse, il sera avantageusement extrait de la phase aqueuse par différentes techniques tels que l'extraction liquide/liquide, l'électrodialyse, traitement sur résines échangeuses d'ions, par exemple.

Dans un second mode de réalisation de l'invention, l'étape d'oxydation des composés α, ω-hydroxycarboxyliques tel que l'acide 6-hydroxycaproïque est réalisée sur la phase aqueuse récupérée après l'étape de refroidissement et décantation du milieu réactionnel d'oxydation, et/ou sur la phase aqueuse de lavage de la phase organique, ainsi que sur les eaux mères récupérées lors de la cristallisation de l'acide dicarboxylique.

Dans ce second mode de réalisation, l'oxydation des composés α, w-hydroxycarboxyliques est réalisée en présence ou en absence de catalyseur par de l'oxygène ou un gaz contenant de l'oxygène, comme l'air, par exemple. Il est également possible d'utiliser d'autres oxydants tels que l'acide nitrique, l'eau oxygénée, l'ozone. La réaction d'oxydation est réalisée à une température comprise entre 50 °C et 150°C et sous une pression d'oxygène comprise entre 0,1 et 30 bar de pression partielle d'oxygène.

Avantageusement, le catalyseur utilisé est un catalyseur hétérogène, par exemple un catalyseur supporté comprenant comme espèce métallique catalytiquement active un composé ou un mélange de composés d'éléments métalliques choisis dans le groupe comprenant Au, Pt, Ru, Cr, Ti, V, Mn, Fe, Co, Zn, Mo, Rh, Pd, Ag, W, Re, Os et Bi. Comme catalyseur particulièrement convenable pour l'invention, on peut citer les catalyseurs à base de platine supporté sur du charbon, de l'alumine, de l'oxyde de titane ou un catalyseur à base de platine et bismuth supporté sur charbon.

Cette opération d'oxydation peut être réalisée sur toutes les phases aqueuses récupérées au cours de l'extraction et la purification de l'acide dicarboxylique, notamment sur les eaux mères de cristallisation. Elle peut également être réalisée simultanément à la séparation par décantation de la phase aqueuse et organique.

Après oxydation, le milieu aqueux récupéré est traité comme précédemment pour extraire les diacides, notamment l'acide adipique.

Avantageusement, le procédé de l'invention peut comprendre une étape d'hydrolyse des esters formés à l'étape d'oxydation. Une telle étape d'hydrolyse est décrite dans le brevet français 2846651, par exemple.

Cette étape d'hydrolyse est avantageusement et préférentiellement mise en oeuvre sur la phase organique récupérée après l'étape de refroidissement et décantation/lavage.

Le procédé de l'invention permet de fabriquer un diacide par oxydation d'un hydrocarbure cyclique par l'oxygène ou un gaz contenant de l'oxygène, avec recyclage de l'hydrocarbure non oxydé, sans accumulation des divers sous produits formés à l'étape d'oxydation. De plus, le ou les diacides récupérés peuvent être facilement purifiés car ils ne sont pas contaminés par certains sous-produits de la réaction d'oxydation de l'hydrocarbure.

D'autres avantages, détails de l'invention apparaîtront plus clairement au vu des exemples.

### Exemple 1 : comparatif

600g d'une solution aqueuse obtenue par séparation du milieu réactionnel provenant de l'oxydation du cyclohexane par de l'air en présence d'acide tertiobutylbenzoïque et d'un catalyseur à base de manganèse et de cobalt tel que décrit dans le brevet français n° 2828194 comprend notamment :
➢ acide adipique : 30%
➢ acide succinique : 2,35%
➢ acide glutarique : 5,60%
➢ acide 6 - hydroxycaproïque : 4,46%

La solution aqueuse obtenue est refroidie afin d'obtenir de l'acide adipique cristallisé. Le solide obtenu par filtration est lavé à l'eau puis repris à chaud, par 300ml d'eau.

La nouvelle solution est refroidie pour permettre la cristallisation de l'acide adipique. Cette opération est répétée une fois.

L'acide hydroxycaproïque est dosé dans l'acide adipique recueilli après chaque cristallisation
➢ 1^{ère} cristallisation : 1986 ppm
➢ 2^{ème} cristallisation : 73 ppm
➢ 3^{ème} cristallisation : 22 ppm

Cet essai montre qu'il est nécessaire de réaliser au moins trois cristallisations successives de l'acide adipique pour obtenir une concentration en acide hydroxycaproïque faible dans l'acide adipique et correspondant aux spécifications requises.

### Exemple 2:

580g d'un milieu réactionnel obtenu lors de l'oxydation du cyclohexane par de l'air en présence d'acide tertiobutylbenzoïque et d'un catalyseur à base de manganèse et de cobalt tel que décrit dans le brevet français n° 2828194 sont lavés avec 250 ml d'eau pour extraire les différents composés solubles dans l'eau notamment les acides formés et l'acide 6-hydrocaproïque

La phase aqueuse résultante contient notamment 1% poids d'acide formique, 0.7% poids d'acide succinique, 3.4% poids d'acide glutarique, 7.3% poids d'acide adipique, 1.4% poids d'acide 6-hydroxycaproique(HOCap). 3.65 g de cette phase aqueuse sont chargés dans un autoclave agité par secousse en présence de Pt supporté sur charbon en poudre commercialisé par la société Engelhard (rapport molaire HOCap/ Pt = 15). La réaction a lieu sous 25 bar de pression d'air à 90°C pendant 3 heures. Après analyse par chromatographie, l'essai conduit à une conversion de l'acide 6-hydroxycaproique de 100%, une conversion de l'acide formique de 100% et un rendement réel en acide adipique de 80%, par rapport à l'acide 6-hydroxycaproïque engagé. Le mélange obtenu est traité par les méthodes classiques de cristallisation de l'acide adipique. La teneur en acide 6-hydroxycaproique (HOCap) dans l'acide adipique après une première cristallisation est inférieure à 2 ppm.

### Exemple 3 :

L'exemple 2 est répété mais en remplaçant dans l'étape d'oxydation de l'acide 6-hydroxycaproique, l'air par H₂O₂ et le catalyseur platine supporté par 13 mg d'acide tungstique.

Après chauffage à 20°C pendant 4 heures, 20.4 % de l'acide hydroxycaproique est transformé en acide adipique

### Exempte 4 :

L'exemple 2 est répété mais en remplaçant, dans l'étape d'oxydation de l'acide 6-hydroxycaproique, l'air par une solution à 60% en poids d'acide nitrique et en utilisant comme catalyseur une composition contenant 6000ppm en poids exprimé en cuivre de nitrate de cuivre (Cu (NO₃)₂, 3H₂O), 300 ppm exprimé en vanadium de VO₃NH₄

La réaction est réalisée à 70°C pendant 3 heures. L'acide 6-hydroxycaproique est totalement transformé. Le rendement en acide adipique est de 68% par rapport à l'acide 6-hydroxycaproïque engagé.

### Exemple 5 :

L'exemple 2 est répété mais en remplaçant le catalyseur platine sur charbon par de l'acétate de palladium ajouté à une concentration de 10% en poids.

Le taux de transformation de l'acide 6-hydroxycaproïque est de 100%. Le rendement en acide adipique est de 63% par rapport à l'acide 6-hydrocaproïque engagé.

### Exemple 6

L'exemple 2 est répété mais en remplaçant le catalyseur platine sur charbon par un catalyseur supporté constitué par de l'alumine comme support et une association Ag/Pd comme phase catalytique supportée, la concentration de la phase catalytique exprimée en poids de métal est de 10% en poids par rapport au support alumine

Le taux de transformation de l'acide 6-hydroxycaproïque est de 57% et le rendement en acide adipique est de 59% par rapport à l'acide 6-hydroxycaproïque transformé

### Exemple 7

L'exemple 2 est répété mais en remplaçant le catalyseur platine sur charbon par un catalyseur supporté constitué par du charbon actif comme support et une association Ru/Fe comme phase catalytique supportée, la concentration de la phase catalytique exprimée en poids de métal est de 10% en poids par rapport au support charbon actif.

Le taux de transformation de l'acide 6-hydroxycaproïque est de 86% et le rendement en acide adipique est de 46% par rapport à l'acide 6-hydroxycaproïque transformé.

### Exemple 8

L'exemple 2 est répété mais en remplaçant le catalyseur platine sur charbon par un catalyseur supporté constitué par du graphite comme support et une association Pt/Bi comme phase catalytique supportée, la concentration de la phase catalytique exprimée en poids de métal est de 10% en poids par rapport au support graphite.

Le taux de transformation de l'acide 6-hydroxycaproïque est de 96% et le rendement en acide adipique est de 81% par rapport à l'acide 6-hydroxycaproïque transformé.

### Exemple 9

L'exemple 2 est répété mais en remplaçant le catalyseur platine sur charbon par un catalyseur supporté constitué par de l'alumine comme support et une association Pt/Bi comme phase catalytique supportée, la concentration de la phase catalytique exprimée en poids de métal est de 10% en poids par rapport au support alumine.

Le taux de transformation de l'acide 6-hydroxycaproïque est de 82% et le rendement en acide adipique est de 69% par rapport à l'acide 6-hydroxycaproïque transformé.

### Exemple 10

L'exemple 2 est répété mais en remplaçant le catalyseur platine sur charbon par un catalyseur supporté constitué par de l'oxyde de titane comme support et du platine comme phase catalytique supportée, la concentration de la phase catalytique exprimée en poids de métal est de 10% en poids par rapport au support oxyde de titane.

Le taux de transformation de l'acide 6-hydroxycaproïque est de 100% et le rendement en acide adipique est de 69% par rapport à l'acide 6-hydroxycaproïque transformé.

## Revendications

1. Procédé de fabrication de diacides carboxyliques par oxydation d'un hydrocarbure avec de l'oxygène ou un gaz contenant de l'oxygène, en présence d'un solvant consistant ;
> A réaliser l'oxydation de l'hydrocarbure
> A extraire du milieu réactionnel les diacides formés par une extraction liquide/liquide avec de l'eau ou une solution aqueuse d'acides comme solvant d'extraction.
> A récupérer les diacides formés par cristallisation à partir de la phase aqueuse récupérée en sortie d'extraction liquide/liquide
> A recycler la phase organique récupérée en sortie de l'étape d'oxydation, à l'étape d'oxydation,
**caractérisé en ce qu'**il comprend une étape de transformation, élimination ou extraction des composés α, ω-hydroxycarboxyliques formés à l'étape d'oxydation consistant à oxyder les dits composés hydroxycarboxyliques en diacides.

2. Procédé selon la revendication 1, **caractérisé en ce qu'**il comprend une ou plusieurs étapes de cristallisation des diacides dans une phase aqueuse.

3. Procédé selon la revendication 1 ou 2, **caractérisé en ce que** l'oxydation des composés hydrocarboxyliques est réalisée sur le milieu réactionnel en sortie de réaction d'oxydation.

4. Procédé selon la revendication 3, **caractérisé en ce que** l'oxydation des composés α, ω-hydroxycarboxyliques est réalisée par addition d'un catalyseur dans le réacteur d'oxydation, en fin d'oxydation de l'hydrocarbure.

5. Procédé selon la revendication 3, **caractérisé en ce que** l'oxydation des composés α, ω-hydroxycarboxyliques est réalisée dans un ou plusieurs réacteurs d'oxydation suplémentaires.

6. Procédé selon l'une des revendications 3 à 5, **caractérisé en ce que** le catalyseur est un catalyseur soluble dans le milieu réactionnel.

7. Procédé selon la revendication 6, **caractérisé en ce que** les acides formés sont extraits par extraction liquide/liquide.

8. Procédé selon la revendication 7, **caractérisé en ce que** le solvant d'extraction est l'eau

9. Procédé selon la revendication 1 ou 2, **caractérisé en ce que** l'oxydation des composés α, ω-hydroxycarboxyliques est réalisée sur la ou les phases aqueuses récupérées après l'étape d'extraction liquide/liquide des diacides et/ou les eaux mères de cristallisation des diacides.

10. Procédé selon la revendication 9, **caractérisé en ce que** l'oxydation des composés α, ω-hydroxycarboxyliques est réalisée à une température comprise entre 50°C et 150°C et une pression partielle en oxygène comprise entre 0,1 et 30 bar.

11. Procédé selon la revendication 9 ou 10, **caractérisé en ce que** l'oxydation des composés α, ω-hydroxycarboxyliques est mise en oeuvre en présence d'un catalyseur métallique.

12. Procédé selon la revendication 11, **caractérisé en ce que** le catalyseur métallique est choisi dans le groupe comprenant Cu, Ag, Au, Mg, Ca, Sr, Ba, Zn, Cd, Hg, Al, Sc, ln, TI, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, les lanthanides comme Ce et les combinaisons de ceux-ci

13. Procédé selon la revendication 11 ou 12, **caractérisé en ce que** le catalyseur est un catalyseur supporté comprenant une phase active constituée par un ou plusieurs éléments appartenant au groupe comprenant Cu, Ag, Au, Mg, Ca, Sr, Ba, Zn, Cd, Hg, Al, Sc, In, TI, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, les lanthanides comme Ce et les combinaisons de ceux-ci et un support choisi dans le groupe comprenant l'alumine, la silice, les zéolithes, les charbons.

14. Procédé selon l'une des revendications 12 ou 13, **caractérisé en ce que** les métaux précieux sont choisis dans le groupe comprenant, l'or, le platine, le palladium, le ruthénium, l'argent.

15. Procédé selon l'une des revendications précédentes, **caractérisé en ce que** l'hydrocarbure est choisi dans le groupe comprenant le cyclohexane, le cyclododécane.

16. Procédé selon la revendication 1, **caractérisé en ce que** le solvant est un acide à caractère lipophile.

## Claims

1. Process for the manufacture of dicarboxylic acids by oxidation of a hydrocarbon with oxygen or a gas comprising oxygen in the presence of a solvent which consists:
➢ in oxidizing the hydrocarbon,
➢ in extracting the diacids formed from the reaction medium by liquid/liquid extraction with water or an aqueous solution of acids as extraction solvent,
➢ in recovering the diacids formed by crystallization from the aqueous phase recovered on conclusion of liquid/liquid extraction,
➢ in recycling, to the oxidation stage, the organic phase recovered on conclusion of the oxidation stage,
**characterized in that** it comprises a stage of conversion, removal or extraction of the α,ω-hydroxycarboxylic compounds formed in the oxidation stage which consists in oxidizing said hydroxycarboxylic compounds to give diacids.

2. Process according to Claim 1, **characterized in that** it comprises one or more stages of crystallization of the diacids from an aqueous phase.

3. Process according to Claim 1 or 2, **characterized in that** the oxidation of the hydroxycarboxylic compounds is carried out on the reaction medium on conclusion of the oxidation reaction.

4. Process according to Claim 3, **characterized in that** the oxidation of the α,ω-hydroxycarboxylic compounds is carried out by addition of a catalyst to the oxidation reactor at the end of oxidation of the hydrocarbon.

5. Process according to Claim 3, **characterized in that** the oxidation of the α,ω-hydroxycarboxylic compounds is carried out in one or more additional oxidation reactors.

6. Process according to one of Claims 3 to 5, **characterized in that** the catalyst is a catalyst which is soluble in the reaction medium.

7. Process according to Claim 6, **characterized in that** the acids formed are extracted by liquid/liquid extraction.

8. Process according to Claim 7, **characterized in that** the extraction solvent is water.

9. Process according to Claim 1 or 2, **characterized in that** the oxidation of the α,ω-hydroxycarboxylic compounds is carried out on the aqueous phase or phases recovered after the stage of liquid/liquid extraction of the diacids and/or the aqueous mother liquors from crystallization of the diacids.

10. Process according to Claim 9, **characterized in that** the oxidation of the α,ω-hydroxycarboxylic compounds is carried out at a temperature of between 50°C and 150°C and an oxygen partial pressure of between 0.1 and 30 bar.

11. Process according to Claim 9 or 10, **characterized in that** the oxidation of the α,ω-hydrdxycarboxylic compounds is carried out in the presence of a metal catalyst.

12. Process according to Claim 11, **characterized in that** the metal catalyst is chosen from the group consisting of Cu, Ag, Au, Mg, Ca, Sr, Ba, Zn, Cd, Hg, Al, Sc, In, Tl, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, lanthanides, such as Ce, and the combinations of these.

13. Process according to Claim 11 or 12, **characterized in that** the catalyst is a supported catalyst comprising an active phase composed of one or more components belonging to the group consisting of Cu, Ag, Au, Mg, Ca, Sr, Ba, Zn, Cd, Hg, Al, Sc, In, Tl, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, lanthanides, such as Ce, and the combinations of these, and a support chosen from the group consisting of alumina, silica, zeolites and charcoals.

14. Process according to either of Claims 12 and 13, **characterized in that** the precious metals are chosen from the group consisting of gold, platinum, palladium, ruthenium or silver.

15. Process according to one of the preceding claims, **characterized in that** the hydrocarbon is chosen from the group consisting of cyclohexane and cyclododecane.

16. Process according to Claim 1, **characterized in that** the solvent is an acid possessing a lipophilic nature.

## Patentansprüche

1. Verfahren zur Herstellung von Dicarbonsäuren durch Oxidation eines Kohlenwasserstoffs mit Sauerstoff oder einem Sauerstoff enthaltenden Gas in Gegenwart eines Lösungsmittels, bei dem man:
➢ den Kohlenwasserstoff oxidiert,
➢ die gebildeten Disäuren durch Flüssig/Flüssig-Extraktion mit Wasser oder einer wässrigen Säurelösung als Extraktionslösungsmittel aus dem Reaktionsmedium extrahiert,
➢ die gebildeten Disäuren durch Kristallisation aus der nach Abschluss der Flüssig/Flüssig-Extraktion gewonnenen wässrigen Phase gewinnt,
➢ die nach Abschluss des Oxidationsschritts gewonnene organische Phase zum Oxidationsschritt zurückführt,
**dadurch gekennzeichnet, dass** es einen Schritt der Umwandlung, Eliminierung oder Extraktion der im Oxidationsschritt gebildeten α,ω-Hydroxycarbonsäureverbindungen umfasst, bei dem man die Hydroxycarbonsäureverbindungen zu Disäuren oxidiert.

2. Verfahren nach Anspruch 1, **dadurch gekennzeichnet, dass** es einen oder mehrere Schritte der Kristallisation der Disäuren aus einer wässrigen Phase umfasst.

3. Verfahren nach Anspruch 1 oder 2, **dadurch gekennzeichnet, dass** man die Oxidation der Hydroxycarbonsäureverbindungen an dem Reaktionsmedium nach Abschluss der Oxidationsreaktion durchführt.

4. Verfahren nach Anspruch 3, **dadurch gekennzeichnet, dass** man die Oxidation der α,ω-Hydroxycarbonsäureverbindungen durch Zugabe eines Katalysators in den Oxidationsreaktor am Ende der Oxidation des Kohlenwasserstoffs durchführt.

5. Verfahren nach Anspruch 3, **dadurch gekennzeichnet, dass** man die Oxidation der α,ω-Hydroxycarbonsäureverbindungen in einem oder mehreren zusätzlichen Oxidationsreaktoren durchführt.

6. Verfahren nach einem der Ansprüche 3 bis 5, **dadurch gekennzeichnet, dass** es sich bei dem Katalysator um einen in dem Reaktionsmedium löslichen Katalysator handelt.

7. Verfahren nach Anspruch 6, **dadurch gekennzeichnet, dass** man die gebildeten Säuren durch Flüssig/Flüssig-Extraktion extrahiert.

8. Verfahren nach Anspruch 7, **dadurch gekennzeichnet, dass** es sich bei dem Extraktionslösungsmittel um Wasser handelt.

9. Verfahren nach Anspruch 1 oder 2, **dadurch gekennzeichnet, dass** man die Oxidation der α,ω-Hydroxycarbonsäureverbindungen an der nach dem Schritt der Flüssig/Flüssig-Extraktion der Disäuren gewonnenen wässrigen Phase bzw. den nach dem Schritt der Flüssig/Flüssig-Extraktion der Disäuren gewonnenen wässrigen Phasen und/oder den Mutterlaugen der Kristallisation der Disäuren durchführt.

10. Verfahren nach Anspruch 9, **dadurch gekennzeichnet, dass** man die Oxidation der α,ω-Hydroxycarbonsäureverbindungen bei einer Temperatur zwischen 50°C und 150°C und einem Sauerstoff-Partialdruck zwischen 0,1 und 30 bar durchführt.

11. Verfahren nach Anspruch 9 oder 10, **dadurch gekennzeichnet, dass** man die Oxidation der α,ω-Hydroxycarbonsäureverbindungen in Gegenwart eines Metallkatalysators durchführt.

12. Verfahren nach Anspruch 11, **dadurch gekennzeichnet, dass** man den Metallkatalysator aus der Gruppe umfassend Cu, Ag, Au, Mg, Ca, Sr, Ba, Zn, Cd, Hg, Al, Sc, In, Tl, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, Lanthaniden wie Ce und Kombinationen davon auswählt.

13. Verfahren nach Anspruch 11 oder 12, **dadurch gekennzeichnet, dass** es sich bei dem Katalysator um einen Trägerkatalysator mit einer aktiven Phase, die aus einem oder mehreren Elementen aus der Gruppe umfassend Cu, Ag, Au, Mg, Ca, Sr, Ba, Zn, Cd, Hg, Al, Sc, In, Tl, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, Lanthaniden wie Ce und Kombinationen davon besteht, und einem Träger aus der Gruppe umfassend Aluminiumoxid, Siliciumoxid, Zeolithen und Kohlen handelt.

14. Verfahren nach einem der Ansprüche 12 oder 13, **dadurch gekennzeichnet, dass** man die Edelmetalle aus der Gruppe umfassend Gold, Platin, Palladium, Ruthenium und Silber auswählt.

15. Verfahren nach einem der vorhergehenden Ansprüche, **dadurch gekennzeichnet, dass** man den Kohlenwasserstoff aus der Gruppe umfassend Cyclohexan und Cyclododecan auswählt.

16. Verfahren nach Anspruch 1, **dadurch gekennzeichnet, dass** es sich bei dem Lösungsmittel um eine Säure mit lipophilem Charakter handelt.
